# EUROPEAN PATENT APPLICATION

(11) **EP 4 563 570 A1**
(43) Date of publication of application: **04.06.2025**
(21) Application number: 23846496.0
(22) Date of filing: 25.07.2023
(51) Int. Cl.: C07C 255/13, C07H 19/067

(54) **ALKYL HALIDE, ALKYLATING AGENT USING SAME, AND METHOD FOR PRODUCING DERIVATIVE OF NUCLEOSIDE**

(30) Priority: 25.07.2022 JP 2022117801
(71) Applicant: Toray Industries, Inc., Tokyo 103-8666 (JP)
(72) Inventor: KOSHIMOTO Kyohei, Kamakura-shi, Kanagawa 248-8555 (JP)
(74) Representative: Kador & Partner Part mbB
(86) International application number: PCT/JP2023/027116
(87) International publication number: WO 2024/024766

(57) **Abstract**

The purpose of the present invention is to provide an alkylating agent that can alkylate a hydroxyl group at 2' position of a synthetic intermediate for an amidite compound in the presence of a base. The present invention provides an alkyl halide that is represented by general formula (I) and can achieve alkylation in the presence of a base. 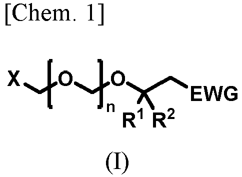 [In the formula, X represents a halogen atom, n represents an integer of 1-5, R¹ and R² are the same or different and represent a methyl group, an ethyl group, or a hydrogen atom, and EWG represents a cyano group or a SO₂R³ group (R³ represents a phenyl group, a C1-10 alkyl group, or a benzyl group which are optionally substituted with a halogen atom, a methyl group, a nitro group, a methoxy group, or a trifluoromethyl group).]

## Description

### Technical Field

The present invention relates to a novel alkyl halide, an alkylating agent using the same, and a production method of a nucleoside derivative.

### Background Art

Examples of methods of synthesizing oligonucleic acids include the phosphate triester method, the H-phosphonate method, and the phosphoramidite method, with the phosphoramidite method being most widely used (Non-Patent Literature 1).

As a raw material for synthesizing nucleic acid by the phosphoramidite method, phosphoramidite (hereinafter referred to as amidite) of nucleoside is used. For example, TBDMS (tert-butyldimethylsilyl), TOM (triisopropylsilyloxymethyl), ACE (bis(2-acetoxyethoxy)methyl), and CEM (cyanoethoxymethyl) (Non-Patent Literature 2) are known as protective groups for the hydroxyl group at the 2' position of amidite. However, these methods of RNA synthesis using amidites having such protective groups are not satisfactory in terms of yield and purity of the resulting RNA.

On the other hand, Patent Literatures 1 to 3 describe amidite (hereinafter referred to as amidite compound) that enables the synthesis of RNA with high purity by using the following group as a protective group for the hydroxyl group at the 2'-position of the amidite. 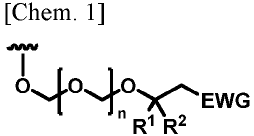 [in the formula, n is an integer of 1 to 5, R¹ and R² are the same or different and each is a methyl group, an ethyl group, or a hydrogen atom, and EWG is a cyano group or a SO₂R³ group (R³ is a phenyl group, an alkyl group having 1 to 10 carbon atoms, or a benzyl group, which may be substituted with a halogen atom(s), a methyl group(s), a nitro group(s), a methoxy group(s), or a trifluoromethyl group(s))].

The amidite compound described in Patent Literature 1 is synthesized by a complicated synthetic method including alkylating a compound of the formula (VI) shown below using an alkylating agent of the formula (V) shown below, followed by three steps.

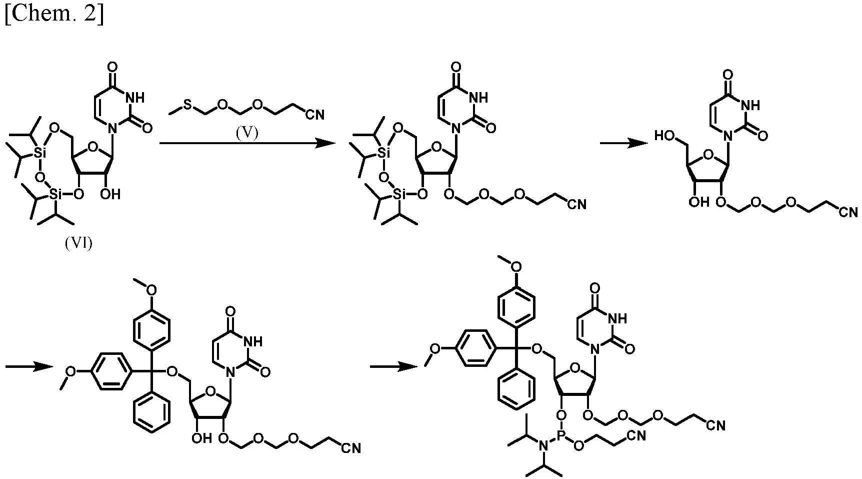

On the other hand, Non-Patent Literature 2 describes as a method of producing CEM amidite, a method of synthesizing in a short step by alkylating a compound of the formula (VIII) shown below using an alkylating agent of the formula (VII) shown below. It is desirable that an amidite compound can also be synthesized in a short step, similarly to the CEM amidite.

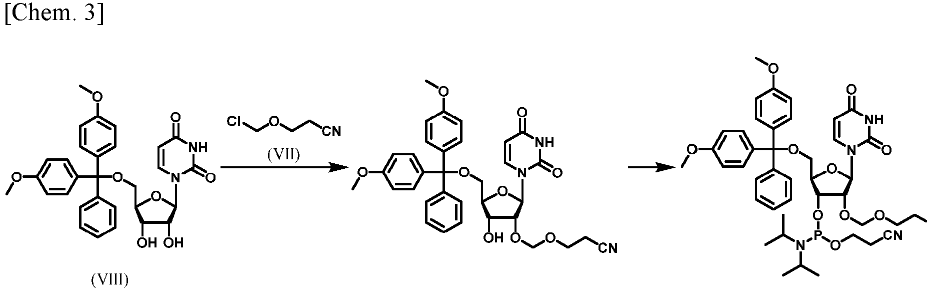

### Citation List

### Patent Literature

Patent Literature 1: WO 2013/027843
Patent Literature 2: WO 2019/208571
Patent Literature 3: WO 2021/079617

### Non-Patent Literature

Non-Patent Literature 1: Nielsen et al., Nucleic Acids Research, 1986, Vol. 14, pp. 7391 - 7403.
Non-Patent Literature 2: Ohgi et al., Orgnic Letters, 2005, Vol. 16, pp.3477 - 3480.

### Summary of Invention

### Technical Problem

However, the presence of an acid is essential for the alkylation of the hydroxyl group at the 2' position of the synthetic intermediate of the amidite compound of the formula (VI) using the alkylating agent of the formula (V). Therefore, the alkylating agent of the formula (V) is not applicable to the alkylation of the compound of the formula (VIII) having a 4,4'-dimethoxytrityl (hereinafter referred to as DMTr) group which is easily decomposed in the presence of an acid.

Therefore, an object of the present invention is to provide an alkylating agent capable of alkylating the hydroxyl group at the 2'-position of a synthetic intermediate of an amidite compound in the presence of a base, in order to synthesize the amidite compound in a short step.

### Solution to Problem

As a result of intensive study to solve the above problems, the present inventors have attained the following inventions (1) through (7).
(1) Alkyl halide of the following general formula (I): 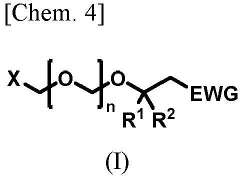 [in the formula, X is a halogen atom, n is an integer of 1 to 5, R¹ and R² are the same or different and each is a methyl group, an ethyl group, or a hydrogen atom, and EWG is a cyano group or a SO₂R³ group (R³ is a phenyl group, an alkyl group having 1 to 10 carbon atoms, or a benzyl group, which may be substituted with a halogen atom(s), a methyl group(s), a nitro group(s), a methoxy group(s), or a trifluoromethyl group(s))]
(2) The alkyl halide according to (1), wherein X is chlorine.
(3) The alkyl halide according to (1) or (2), wherein EWG is a cyano group.
(4) The alkyl halide according to any one of (1) to (3), wherein n is 1.
(5) The alkyl halide according to any one of (1) to (4), wherein the alkyl halide is an alkylating agent.
(6) A production method of a nucleoside derivative of the general formula (III) shown below, the production method including an alkylation step in which alkyl halide of the general formula (I) shown below is reacted with a nucleoside derivative of the general formula (II) shown below in the presence of a base. 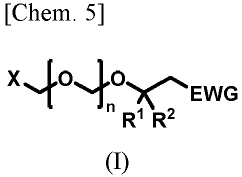 [in the formula, X is a halogen atom, n is an integer of 1 to 5, R¹ and R² are the same or different and each is a methyl group, an ethyl group, or a hydrogen atom, and EWG is a cyano group or a SO₂R³ group (R³ is a phenyl group, an alkyl group having 1 to 10 carbon atoms, or a benzyl group, which may be substituted with a halogen atom(s), a methyl group(s), a nitro group(s), a methoxy group(s), or a trifluoromethyl group(s))] 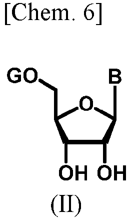 [in the formula, G is a protective group for a hydroxyl group, and B is a nucleobase which may be protected and/or substituted] 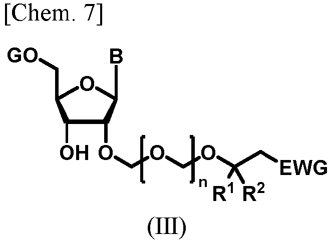 [in the formula, G is a protective group for a hydroxyl group, B is a nucleobase which may be protected and/or substituted, n is an integer of 1 to 5, R¹ and R² are the same or different and each is a methyl group, an ethyl group, or a hydrogen atom, and EWG is a cyano group or a SO₂R³ group (R³ is a phenyl group, an alkyl group having 1 to 10 carbon atoms, or a benzyl group, which may be substituted with a halogen atom(s), a methyl group(s), a nitro group(s), a methoxy group(s), or a trifluoromethyl group(s))]
(7) The production method according to (6), wherein G is a trityl group, a 4-methoxytrityl group, or a DMTr group.

### Advantageous Effects of Invention

According to the present invention, a nucleoside derivative of the general formula (II) shown below can be alkylated in the presence of a base using an alkyl halide of the general formula (I) shown below as an alkylating agent to obtain a nucleoside derivative of the general formula (III) shown below. 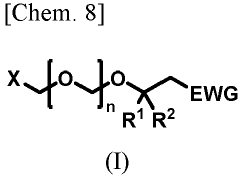 [in the formula, X is a halogen atom, n is an integer of 1 to 5, R¹ and R² are the same or different and each is a methyl group, an ethyl group, or a hydrogen atom, and EWG is a cyano group or a SO₂R³ group (R³ is a phenyl group, an alkyl group having 1 to 10 carbon atoms, or a benzyl group, which may be substituted with a halogen atom(s), a methyl group(s), a nitro group(s), a methoxy group(s), or a trifluoromethyl group(s))] 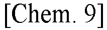 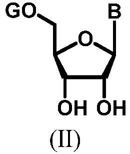 [in the formula, G is a protective group for a hydroxyl group, and B is a nucleobase which may be protected and/or substituted] 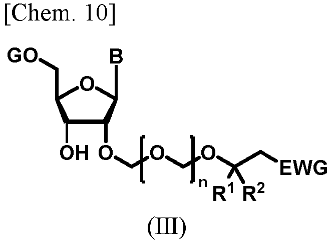 [in the formula, G is a protective group for a hydroxyl group, B is a nucleobase which may be protected and/or substituted, n is an integer of 1 to 5, R¹ and R² are the same or different and each is a methyl group, an ethyl group, or a hydrogen atom, and EWG is a cyano group or a SO₂R³ group (R³ is a phenyl group, an alkyl group having 1 to 10 carbon atoms, or a benzyl group, which may be substituted with a halogen atom(s), a methyl group(s), a nitro group(s), a methoxy group(s), or a trifluoromethyl group(s))]

### Description of Embodiments

The present invention will be described in detail below.

The alkyl halide of the present invention is characterized by the following general formula (I): 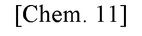 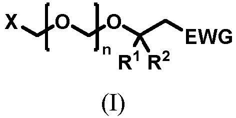 [in the formula, X is a halogen atom, n is an integer of 1 to 5, R¹ and R² are the same or different and each is a methyl group, an ethyl group, or a hydrogen atom, and EWG is a cyano group or a SO₂R³ group (R³ is a phenyl group, an alkyl group having 1 to 10 carbon atoms, or a benzyl group, which may be substituted with a halogen atom(s), a methyl group(s), a nitro group(s), a methoxy group(s), or a trifluoromethyl group(s))]

The alkyl halide of the above general formula (I) may be not only a single stereoisomer but also a mixture of stereoisomers such as a racemate (for example, a mixture of enantiomers).

Stereoisomers refer to compounds that have the same chemical structure but different arrangements in three-dimensional space. Examples include conformational isomers, rotational isomers, tautomers, enantiomers, and diastereomers.

Examples of halogen atoms include fluorine, chlorine, bromine, and iodine.

The alkyl group may be either linear or branched, and is preferably an alkyl group having 1 to 10 carbon atoms, more preferably an alkyl group having 1 to 6 carbon atoms. Examples of the alkyl group include, but are not limited to, a methyl group, an ethyl group, an n-propyl group, an isopropyl group, an n-butyl group, an isobutyl group, a tert-butyl group, an n-pentyl group, an isopentyl group, and a hexyl group.

In the above general formula (I), X is preferably chlorine, bromine, or iodine, more preferably chlorine, and may be an alkyl chloride of the following general formula (I'): 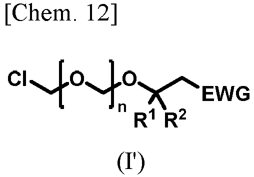 [in the formula, n is an integer of 1 to 5; R¹ and R² are the same or different and each is a methyl group, an ethyl group, or a hydrogen atom, and EWG is a cyano group or a SO₂R³ group (R³ is a phenyl group, an alkyl group having 1 to 10 carbon atoms, or a benzyl group, which may be substituted with a halogen atom(s), a methyl group(s), a nitro group(s), a methoxy group(s), or a trifluoromethyl group(s))].

In the above general formula (I), n is preferably an integer from 1 to 4, more preferably 1.

In the above general formula (I), R¹ and R² are preferably each independently a methyl group or a hydrogen atom.

In the above general formula (I), as a preferred aspect of the combination of X, n, R¹, R², and EWG, a combination in which X is chlorine, n is 1, R¹ and R² are both hydrogen atoms, and EWG is a cyano group, or a combination in which X is chlorine, n is 1, R¹ is a methyl group, R² is a hydrogen atom, and EWG is a cyano group is suitable.

### (Production method of alkyl halide of general formula (I))

As an example of the production method of the alkyl halide of the general formula (I), a production method of the alkyl chloride of the general formula (I') is shown below.

The alkyl chloride of the above general formula (I') can be produced, for example, but not limited to, by reacting a compound of the general formula (IV) shown below with sulfuryl chloride in a solvent according to the following scheme 1. 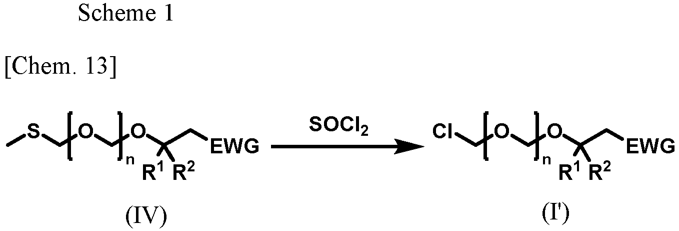 [in the formula, n is an integer of 1 to 5; R¹ and R² are the same or different and each is a methyl group, an ethyl group, or a hydrogen atom, and EWG is a cyano group or a SO₂R³ group (R³ is a phenyl group, an alkyl group having 1 to 10 carbon atoms, or a benzyl group, which may be substituted with a halogen atom(s), a methyl group(s), a nitro group(s), a methoxy group(s), or a trifluoromethyl group(s))].

The molar equivalent of sulfuryl chloride is preferably 0.5 to 10 molar equivalents, more preferably 0.5 to 2 molar equivalents, relative to the compound of the above general formula (IV).

Examples of the solvent used in the production of the alkyl chloride of the above general formula (I') includes, but is not limited to, chloroform, dichloromethane, and dichloroethane.

The amount of solvent used in the production of alkyl chloride of the above general formula (I') is preferably 1 to 100 times by weight relative to the compound of the above general formula (IV).

The reaction time for production of the alkyl chlorides of the above general formula (I') can be appropriately set, but typically may be 1 hour or more, for example, from 1 to 2 hours.

The reaction temperature for production of the alkyl chlorides of the above general formula (I') can be appropriately set and may range from 10°C to 100°C. Usually, room temperature (about 10°C to about 35°C) is preferred.

Alkyl halide of the above general formula (I) can be used as an alkylating agent.

In a preferred embodiment, the alkyl chloride of the above general formula (I') may be alkyl chloride of the following formula (I'-1) or the following formula (I'-2):

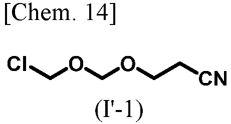

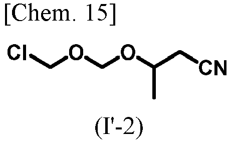

Further, the present invention provides a method of producing a nucleoside derivative of the general formula (III) shown below, the method including an alkylation step in which an alkyl halide of the above general formula (I) is reacted with a nucleoside derivative of the general formula (II) shown below in the presence of a base. The steps of the production method are shown below. 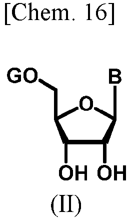 [in the formula, G is a protective group for a hydroxyl group, and B is a nucleobase which may be protected and/or substituted] 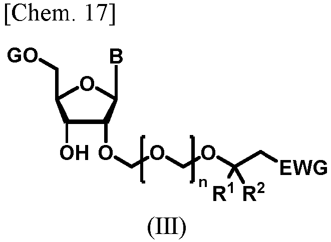 [in the formula, G is a protective group for a hydroxyl group, B is a nucleobase which may be protected and/or substituted, n is an integer of 1 to 5, R¹ and R² are the same or different and each is a methyl group, an ethyl group, or a hydrogen atom, and EWG is a cyano group or a SO₂R³ group (R³ is a phenyl group, an alkyl group having 1 to 10 carbon atoms, or a benzyl group, which may be substituted with a halogen atom(s), a methyl group(s), a nitro group(s), a methoxy group(s), or a trifluoromethyl group(s))]

Further, a nucleoside derivative of the above general formula (III) is obtained by an alkylation step (Scheme 2 shown below) in which alkyl halide of the above general formula (I) is reacted with a nucleoside derivative of the above general formula (II) in the presence of a base. 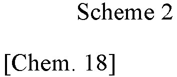 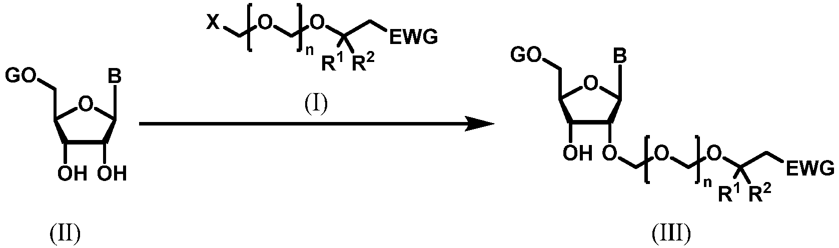 [in the formula, G is a protective group for a hydroxyl group, B is a nucleobase which may be protected and/or substituted, X is a halogen atom, n is an integer of 1 to 5, R¹ and R² are the same or different and each is a methyl group, an ethyl group, or a hydrogen atom, and EWG is a cyano group or a SO₂R³ group (R³ is a phenyl group, an alkyl group having 1 to 10 carbon atoms, or a benzyl group, which may be substituted with a halogen atom(s), a methyl group(s), a nitro group(s), a methoxy group(s), or a trifluoromethyl group(s))]

The nucleoside derivatives of the above general formulas (II) and (III) may be not only a single stereoisomer but also a mixture of stereoisomers such as a racemate (for example, a mixture of enantiomers).

The nucleoside derivative of the above general formula (II) may be a commercially available product, or may be synthesized by a known method or a method similar thereto.

In the above general formulas (II) and (III), G is a protective group, for example, a functional group that converts a hydroxyl group to an inactive one, and may be a known protective group for a hydroxyl group. Regarding the protective group for a hydroxyl group, for example, the description in the literature (Protective Groups in Organic Synthesis 4th edition, Greene et al., 2007, John Wiley & Sons, Inc.) can be incorporated herein. Examples of G include, but not limited to, a tert-butyldimethylsilyl group, a bis(2-acetoxyethyloxy)methyl group, a triisopropylsilyloxymethyl group, a 1-(2-cyanoethoxy)ethyl group, a 2-cyanoethoxymethyl group, a 2-cyanoethyl group, a trisulfonylethoxymethyl group, a trityl group, a 4-methoxytrityl group, or a DMTr group. G is preferably a trityl group, a 4-methoxytrityl group, or a DMTr group.

In the above general formulas (II) and (III), B is a nucleobase which may be protected and/or substituted. Examples of the nucleobase include, but are not limited to, adenine, cytosine, guanine, uracil, thymine, 5-methylcytosine, pseudouracil, and 1-methylpseudouracil. Furthermore, examples of the substituent on the nucleobase include, but are not limited to, a halogen atom, an acyl group, an alkyl group, an arylalkyl group, an alkoxy group, an alkoxyalkyl group, a cyanoalkyl group, a hydroxy group, a hydroxymethyl group, an acyloxymethyl group, an amino group, a monoalkylamino group, a dialkylamino group, a carboxy group, a cyano group, and a nitro group, as well as combinations of two or more of these substituents.

When a nucleobase has an exocyclic amino group, the protective group for the amino group can be a known protective group used in the field of nucleic acid chemistry, and examples thereof include, but are not limited to, a methyl group, a benzoyl group, a 4-methoxybenzoyl group, an acetyl group, a propionyl group, a butyryl group, an isobutyryl group, a phenylacetyl group, a phenoxyacetyl group, a 4-tert-butylphenoxyacetyl group, a 4-isopropylphenoxyacetyl group, a dimethylaminomethylene group, and combinations of two or more of these protective groups.

Examples of the base used in the alkylation step include, but are not limited to, a tertiary amine, a pyridine derivative, an aniline derivative, or an inorganic base.

Examples of the tertiary amine include, but are not limited to, triethylamine, tri-n-butylamine, diisopropylethylamine, dicyclohexylmethylamine, 1,2,2,6,6-pentamethylpiperidine, and 1,2,2,6,6-pentamethyl-4-piperidyl methacrylate.

Examples of the pyridine derivative include, but are not limited to, 2,6-lutidine, 2,6-di-tert-butylpyridine, 2,6-dimethoxypyridine, 2,6-dichloropyridine, and 2,6-dibromopyridine.

Examples of the aniline derivative include, but are not limited to, 2,6-dimethylaniline, 2,6-diisopropylaniline, N,N-dimethylaniline, N,N-diethylaniline, and N,N,2-trimethylaniline.

The molar equivalent of the base used in the alkylation step is preferably 1 to 10 molar equivalents, more preferably 2 to 4 molar equivalents, relative to the nucleoside derivative of the above general formula (II).

An additive may be used in the alkylation step, and examples of the additive include, but are not limited to, a tin compound, a boron compound, and an antimony compound.

Examples of the tin compound include, but are not limited to, n-butyltin trichloride, dimethyltin dichloride, di-n-butyltin dichloride, di-tert-butyltin dichloride, diphenyltin dichloride, tri-n-butyltin chloride, and di-n-butyltin oxide.

Examples of the boron compound include, but are not limited to, phenylboronic acid, 4-trifluoromethylphenylboronic acid, 4-chlorophenylboronic acid, 4-methoxyphenylboronic acid, 2-trifluoromethylphenylboronic acid, 2-chlorophenylboronic acid, 2-biphenylboronic acid, 2,6-dichlorophenylboronic acid, 2-furylboronic acid, 3-pyridylboronic acid, 4-pyridylboronic acid, diphenylborinic acid, 2-aminoethyl diphenylborinate, dimesitylborinic acid, and triphenylborane.

Examples of the antimony compound include, but are not limited to, triphenylantimony dichloride, triphenylantimony oxide, and tetraphenylantimony bromide.

The molar equivalent of the additive used in the alkylation step is preferably 0.1 to 10 molar equivalents, more preferably 0.1 to 2 molar equivalents, relative to the nucleoside derivative of the above general formula (II).

Solvents may be used in the alkylation step. Examples of the solvent include, but are not limited to, toluene, xylene, dichloromethane, chloroform, dichloroethane, methyl tert-butyl ether, cyclopentyl methyl ether, diethyl ether, dimethoxyethane, tetrahydrofuran, 1,4-dioxane, methyl ethyl ketone, methyl isobutyl ketone, acetone, methyl acetate, ethyl acetate, dimethylformamide, dimethylacetamide, N-methyl pyrrolidone, 1,3-dimethyl-2-imidazolidinone, N,N'-dimethylpropyleneurea, acetonitrile, propionitrile, dimethyl sulfoxide, and sulfolane.

The amount of solvent used in the alkylation step is preferably 1 to 100 times by weight relative to the nucleoside derivative of the above general formula (II).

The reaction time for the alkylation step can be appropriately set, but may be typically 2 hours or more, for example, 2 to 24 hours.

The reaction temperature for the alkylation step can be appropriately set, and may range from 10°C to 100°C, for example. Usually, room temperature (about 10°C to about 35°C) is preferred.

### Examples

Hereinafter, the present invention will be more specifically described with reference to Examples. However, the technical scope of the present invention is not limited to these examples.

### (Example 1)

Synthesis of 2-cyanoethoxymethoxymethyl (hereinafter referred to as "EMM") chloride (Scheme 3 below): Scheme 3

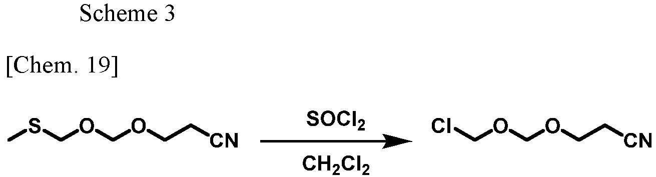

EMM thiomethyl (10.0 g, 62.0 mmol) was dissolved in dichloromethane (100 mL), and then sulfuryl chloride (10.5 g, 78.0 mmol) was added. The reaction solution was stirred at room temperature for 1 hour, and the solvent was then distilled off under reduced pressure. The resulting residue was purified by distillation under reduced pressure (1 mmHg, 94°C) to obtain EMM chloride (7.01 g, yield 76%) of the following formula (I'-1) as a yellow liquid.

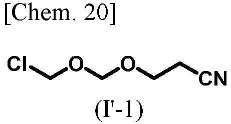

¹H-NMR(400MHz, CDCl₃): δ 5.54 (s, 2H), 4.92 (s, 2H), 3.82 (t, *J =* 6.2 Hz, 2H), 2.66 (t, *J =* 6.2 Hz, 2H).

### (Example 2)

Synthesis of 1-cyanopropane-2-yl-oxymethoxymethyl (hereinafter referred to as "PMM") chloride (Scheme 4 below): Scheme 4

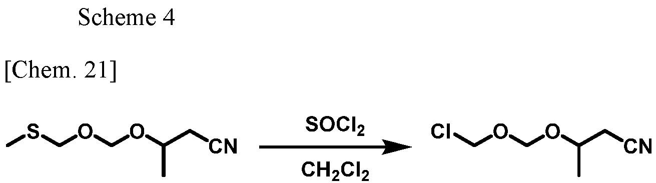

PMM thiomethyl (336 mg, 1.92 mmol) was dissolved in dichloromethane (1.70 mL), and then sulfuryl chloride (311 mg, 2.30 mmol) was added. The reaction solution was stirred at room temperature for 1 hour, and the solvent was then distilled off under reduced pressure. The resulting residue was purified by distillation under reduced pressure (4 mmHg, 110°C) to obtain PMM chloride (189 mg, yield 60%) of the following formula (I'-2) as a yellow liquid.

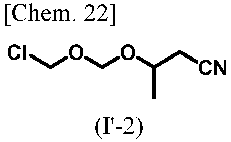

¹H-NMR (400 MHz, CDCl₃): δ 5.60 (d, *J =* 5.9 Hz, 1H), 5.54 (d, *J =* 5.9 Hz, 1H), 4.97 (d, *J =* 7.3 Hz, 1H), 4.90 (d, *J =* 7.3 Hz, 1H), 4.07 - 4.00 (m, 1H), 2.61 (dd, *J =* 16.7, 5.3 Hz, 1H), 2.55 (dd, *J =* 16.7, 6.2 Hz, 2H), 1.35 (d, *J =* 5.9 Hz, 3H).

### (Example 3)

### Synthesis of DMTr-EMM-uridine (Scheme 5 below):

Scheme 5

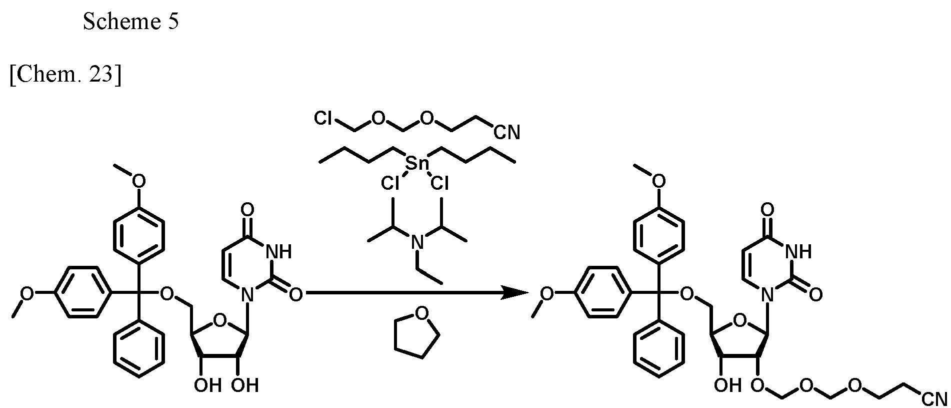

DMTr-uridine (50.0 mg, 0.0915 mmol) was dissolved in tetrahydrofuran (0.500 mL), and then diisopropylethylamine (47.3 mg, 0.366 mmol) and di-n-butyltin dichloride (30.6 mg, 0.101 mmol) were added. The reaction solution was stirred at room temperature for 30 minutes before EMM chloride (16.4 mg, 0.110 mmol) was added. The reaction solution was further stirred at room temperature for 4 hours, and then purified by column chromatography (developing solvent: chloroform/methanol = 100/0 to 90/10) using amino silica gel as a filler to obtain DMTr-EMM-uridine (39.1 mg, yield 65%).

### Industrial Applicability

By using the alkyl halide of the present invention as an alkylating agent, the hydroxyl group at the 2'-position of a synthetic intermediate of an amidite compound can be alkylated in the presence of a base.

## Claims

1. Alkyl halide of the following general formula (I): 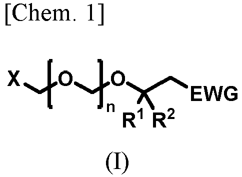 [in the formula, X is a halogen atom, n is an integer of 1 to 5, R¹ and R² are the same or different and each is a methyl group, an ethyl group, or a hydrogen atom, and EWG is a cyano group or a SO₂R³ group (R³ is a phenyl group, an alkyl group having 1 to 10 carbon atoms, or a benzyl group, which may be substituted with a halogen atom(s), a methyl group(s), a nitro group(s), a methoxy group(s), or a trifluoromethyl group(s))].

2. The alkyl halide according to claim 1, wherein X is chlorine.

3. The alkyl halide according to claim 1 or 2, wherein EWG is a cyano group.

4. The alkyl halide according to any one of claims 1 to 3, wherein n is 1.

5. The alkyl halide according to any one of claims 1 to 4, wherein the alkyl halide is an alkylating agent.

6. A production method of a nucleoside derivative of the general formula (III) shown below,
the production method comprising an alkylation step in which alkyl halide of the general formula (I) shown below is reacted with a nucleoside derivative of the general formula (II) shown below in the presence of a base. 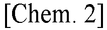 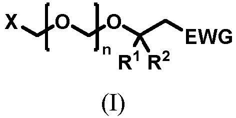 [in the formula, X is a halogen atom, n is an integer of 1 to 5, R¹ and R² are the same or different and each is a methyl group, an ethyl group, or a hydrogen atom, and EWG is a cyano group or a SO₂R³ group (R³ is a phenyl group, an alkyl group having 1 to 10 carbon atoms, or a benzyl group, which may be substituted with a halogen atom(s), a methyl group(s), a nitro group(s), a methoxy group(s), or a trifluoromethyl group(s))] 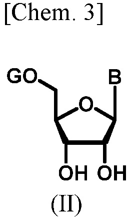 [in the formula, G is a protective group for a hydroxyl group, and B is a nucleobase which may be protected and/or substituted] 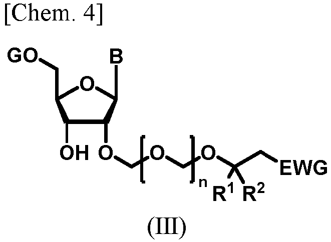 [in the formula, G is a protective group for a hydroxyl group, B is a nucleobase which may be protected and/or substituted, n is an integer of 1 to 5, R¹ and R² are the same or different and each is a methyl group, an ethyl group, or a hydrogen atom, and EWG is a cyano group or a SO₂R³ group (R³ is a phenyl group, an alkyl group having 1 to 10 carbon atoms, or a benzyl group, which may be substituted with a halogen atom(s), a methyl group(s), a nitro group(s), a methoxy group(s), or a trifluoromethyl group(s))]

7. The production method according to claim 6, wherein G is a trityl group, a 4-methoxytrityl group, or a 4,4'-dimethoxytrityl group.
